# FASCICULE DE BREVET EUROPEEN

(11) **EP 1 420 028 B1**
(45) Date de publication et mention de la délivrance du brevet: **21.02.2007**
(21) Numéro de dépôt: 03292864.0
(22) Date de dépôt: 19.11.2003
(51) Int. Cl.: C07K 5/06, C07C 309/73, C07C 229/36

(54) **Pocédé de synthèse du perindopril et de ses sels pharmaceutiquement acceptables**
Verfahren für die Synthese von Perindopril und seiner pharmazeutischen annehmbaren Salzen
Method for synthesis of perindopril and its pharmaceutically acceptable salts

(43) Date de publication de la demande: 19.05.2004
(73) Titulaire: Les Laboratoires Servier, 92415 Courbevoie Cédex (FR)
(72) Inventeur: Dubuffet, Thierry, 76190 Autretot (FR); Langlois, Pascal, 76210 Saint Jean de la Neuville (FR)

(56) Documents cités:
- EP-A- 0 308 341
- SEBLE WAGAW ET AL.: "Palladium-Catalyzed Coupling of Optically active Amines with Aryl Bromides" JOURNAL OF THE AMERICAN CHEMICAL SOCIETY, AMERICAN CHEMICAL SOCIETY, WASHINGTON, DC, US, vol. 119, no. 36, pages 8451-8458, XP002274577 ISSN: 0002-7863

## Description

La présente invention concerne un procédé de synthèse du perindopril de formule (I) : 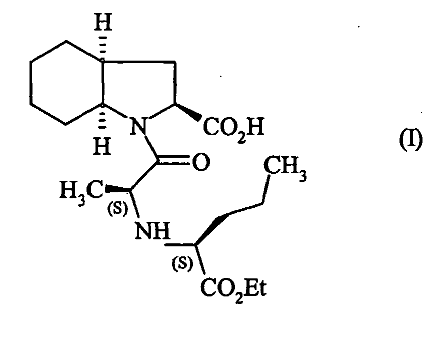 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2*S,* 3a*S*, 7a*S*)-octahydroindole 2-carboxylique avec l'ester éthylique de la N-[(*S*)-1-carboxybutyl]-(*S*)-alanine, suivie de la déprotection du groupement carboxylique de l'hétérocycle par hydrogénation catalytique.

La demanderesse a présentement mis au point un nouveau procédé de synthèse du perindopril à partir de matières premières aisément accessibles.

Plus spécifiquement, la présente invention concerne un procédé de synthèse du perindopril, et de ses sels pharmaceutiquement acceptables, caractérisé en ce que l'on fait réagir le composé de formule (II), de configuration (S) : 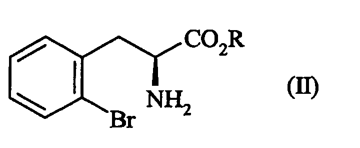 dans laquelle R représente un atome d'hydrogène ou un groupement protecteur de la fonction acide,
avec un composé de formule (III), de configuration (R) : 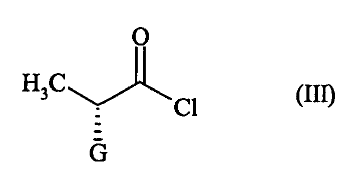 dans laquelle G représente un atome de chlore ou de brome ou un groupement hydroxy, p-toluènesulfonyloxy, méthanesulfonyloxy ou trifluorométhanesulfonyloxy,
en présence de base,
pour conduire au composé de formule (IV) : 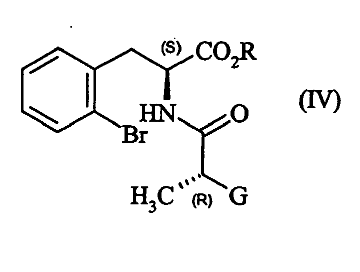 dans laquelle R et G sont tels que définis précédemment,
que l'on soumet à une réaction de couplage intramoléculaire, pour conduire au composé de formule (V) : 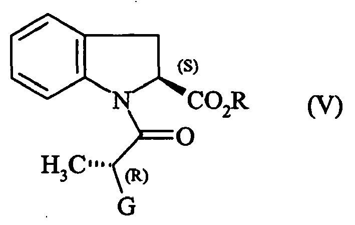 dans laquelle R et G sont tels que définis précédemment,
que l'on met en réaction avec le composé de formule (VI) : 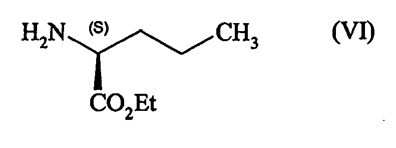 pour conduire au composé de formule (VII) : 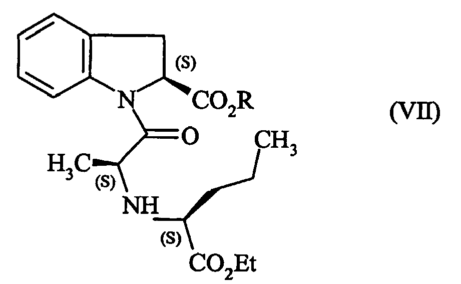 dans laquelle R est tel que défini précédemment,
que l'on soumet à une réaction d'hydrogénation catalytique, pour conduire, après déprotection le cas échéant, au composé de formule (I).

Parmi les groupements protecteurs de la fonction acide, on peut citer à titre non limitatif les groupements benzyle et alkyle (C₁-C₆) linéaire ou ramifié.

Parmi les bases utilisables pour la réaction entre les composés de formules (II) et (III), on peut citer à titre non limitatif les amines organiques telles que la triéthylamine, la pyridine ou la diisopropyléthylamine, et les bases minérales telles que NaOH, KOH, Na₂CO₃, K₂CO₃, NaHCO₃ ou KHCO₃.

La réaction de couplage intramoléculaire est préférentiellement effectuée, soit en présence d'une base et d'un catalyseur à base de palladium, soit à l'aide d'hydrure de sodium et d'iodure de cuivre (I) ou de bromure de cuivre (I).
Les catalyseurs à base de palladium préférentiellement utilisés pour cette réaction de couplage sont les catalyseurs à base de palladium et d'une arylphosphine ou d'une bis-phosphine.
Parmi ces catalyseurs, on peut citer à titre non limitatif Pd(0)/PPh₃, Pd(0)/P(*o*-tolyl)₃, Pd(0)/P(1-naphtyl)₃, Pd(0)/P(*o*-méthoxyphényl)₃, Pd₂(dba)₃/PPh₃, Pd₂(dba)₃/P(*o*-tolyl)₃, Pd₂(dba)₃/P(1-naphtyl)₃, Pd₂(dba)₃/P(*o*-méthoxyphényl)₃, Pd₂(dba)₃/P(2-furyl)₃, Pd₂(dba)₃/dppp, Pd₂(dba)₃/(±)-BINAP et (DPPF)PdCl₂.CH₂Cl₂/DPPF,
étant entendu que par BINAP, on entend 2,2'-bis(diphénylphosphino)-1,1'-binaphtyle, par dba, on entend dibenzylidèneacétone,
par DPPF, on entend 1,1'-bis(diphénylphosphino)ferrocène,
et par dppp, on entend 1,3-bis(diphénylphosphino)propane.

Parmi les bases utilisables pour la réaction de couplage en présence d'un catalyseur à base de palladium, on peut citer à titre non limitatif Cs₂CO₃, NaOtBu, Na₂CO₃, NaOAc et KOAc.

Lorsque G représente un atome de chlore ou de brome, ou un groupement p-toluènesulfonyloxy, méthanesulfonyloxy ou trifluorométhanesulfonyloxy, la réaction entre les composés de formules (V) et (VI) est préférentiellement effectuée en présence d'une base, de préférence une amine organique telle que la triéthylamine, la pyridine ou la diisopropyléthylamine ou une base minérale telle que Na₂CO₃, K₂CO₃, NaHCO₃ ou KHCO₃.

Lorsque G représente un groupement hydroxy, la réaction entre les composés de formules (V) et (VI) est préférentiellement effectuée en présence d'un réactif d'activation tel que l'iodure de N-méthyl-N-phényl-aminotriphénylphosphonium, ou, lorsque R est différent de l'atome de hydrogène, par réaction de Mitsunobu.

Les composés de formule (IV) sont des produits nouveaux, utiles comme intermédiaires de synthèse dans l'industrie chimique ou pharmaceutique, notamment dans la synthèse du perindopril, et font à ce titre partie intégrante de la présente invention.

Les composés de formule (II) peuvent être préparés selon le procédé décrit dans la publication *J. Am. Chem. Soc.* **1994**, *116*, 10847-10848.

### EXEMPLE 1 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-3-(2-Bromophényl)-2-{[(2R)-2-bromopropanoyl]amino}-propanoate de benzyle

Dans un réacteur, charger 25,7 g de (*S*)-2-bromophénylalaninate de benzyle et 150 ml de dichlorométhane, puis amener la température du mélange réactionnel à 0°C et ajouter 20 ml de diisopropyléthylamine, puis 13,2 g de chlorure de (2R)-2-bromopropionyle. Amener ensuite le mélange à température ambiante. Après 1h d'agitation à cette température, laver le mélange à l'eau puis avec une solution diluée d'acide acétique, et évaporer les solvants, pour conduire au produit du titre.

### Stade B : (2S)-1-[(2R)-2-Bromopropanoyl]-2-indolinecarboxylate de benzyle :

Dans un réacteur, charger 15,5 g du composé obtenu au stade précédent en solution dans le toluène, 1,57 g de Pd₂(dba)₃, 1,83 g de P(o-tolyl)₃ et 21,5 g de Cs₂CO₃. Amener ensuite le mélange réactionnel à 100°C. Après 15h d'agitation à cette température, le mélange est ramené à température ambiante et purifié par chromatographie sur silice, pour conduire au produit du titre.

### Stade C: (2S)-I-((2S)-2-{[(1 S)-l-(Ethoxycarbonyl)butyl]amino}propanoyl)-2-indolinecarboxylate de benzyle :

Dans un réacteur, charger 12,3 g de (2*S*)-2-aminopentanoate d'éthyle, 16 ml de triéthylamine et 16 ml d'acétonitrile, puis amener le mélange à 60°C, ajouter lentement une solution de 19,4 g du composé obtenu au stade précédent en solution dans le dichlorométhane, et chauffer au reflux pendant 4h. Après retour à température ambiante, laver le mélange à l'eau et avec une solution diluée d'acide acétique, puis évaporer les solvants, pour conduire au produit du titre.

### Stade D : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 20 g du composé obtenu dans le stade précédent, en solution dans l'acide acétique, puis 0,5 g de Pd/C à 10 %. Hydrogéner sous pression de 0,5 bars entre 15 et 30°C, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant, pour conduire au produit du titre avec une pureté énantiomérique de 99 %.

### Stade E : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le précipité obtenu dans le stade précédent (20 g) est mis en solution dans 280 ml d'acétate d'éthyle, puis 4 g de tert-butylamine et 40 ml d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

### EXEMPLE 2 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : Acide (2S)-3-(2-bromophényl)-2-{[(2R)-2-bromopropanoyl]amino}-propanoïque

Dans un réacteur, charger 28,8 g de (S)-2-bromophénylalanine, 7,5 ml d'eau et 15 ml de toluène, puis amener le mélange entre 0 et 5°C et ajouter 25 ml de soude 5M, puis une solution de 20,2 g de chlorure de (2R)-2-bromopropionyle dans le toluène, tout en maintenant la température en dessous de 10°C, et le pH du milieu à 10 par ajout de soude 5M. Après 1h d'agitation supplémentaire à 10°C, ajouter de l'acide chlorhydrique concentré pour amener le pH du mélange à 6.
Séparer la phase toluénique, puis ajouter à la phase aqueuse de l'acide chlorhydrique concentré pour amener le pH à 2.
Le précipité formé est alors filtré et séché, pour conduire au produit du titre.

### Stade B : identique au stade B de l'exemple 1.

### Stade C : Acide (2S)-1-((2S)-2-{[(1S)-1-(éthoxycarbonyl)-butyl]-amino}-propanoyl)-2-indolinecarboxylique :

Dans un réacteur, charger 10,5 g de (2*S*)-2-aminopentanoate d'éthyle, 13,5 ml de triéthylamine et 13,5 ml d'acétonitrile, puis amener le mélange à 60°C et ajouter lentement une solution de 19,3 g du composé obtenu au stade précédent dans 130 ml de dichlorométhane, puis chauffer au reflux pendant 4h. Après retour à température ambiante, laver le mélange à l'eau et avec une solution diluée d'acide acétique, puis évaporer les solvants, pour conduire au produit du titre.

### Stades D et E : identiques aux stades D et E de l'exemple 1.

### EXEMPLE 3 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-3-(2-Bromophényl)-2-{[(2R)-2-(p-toluènesulfonyloxy)-propanoyl]-amino}-propanoate de benzyle :

Dans un réacteur, charger 25,7 g de (*R*)-2-bromophénylalaninate de benzyle, 150 ml de dichlorométhane, puis amener la température du mélange réactionnel à 0°C et ajouter 20 ml de diisopropyléthylamine, puis 20,2 g de chlorure de (1*R*)-2-chloro-1-méthyl-2-oxoéthyl-p-toluènesulfonate. Amener ensuite le mélange à température ambiante. Après 1h d'agitation à cette température, laver le mélange à l'eau. Les solvants sont ensuite évaporés, pour conduire au produit du titre.

*Stades B à E : identiques aux stades B à E de l'exemple 1*.

## Revendications

1. Procédé de synthèse du perindopril de formule (I) : 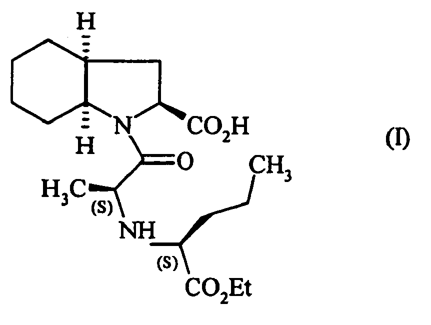 et de ses sels pharmaceutiquement acceptables,
**caractérisé en ce que** l'on fait réagir le composé de formule (II), de configuration (S) : 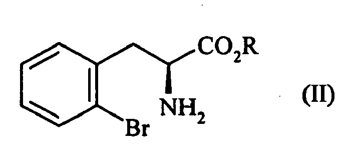 dans laquelle R représente un atome d'hydrogène ou un groupement protecteur de la fonction acide,
avec un composé de formule (III), de configuration (R) : 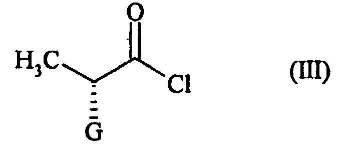 dans laquelle G représente un atome de chlore ou de brome ou un groupement hydroxy, p-toluènesulfonyloxy, méthanesulfonyloxy ou trifluorométhanesulfonyloxy,
en présence de base,
pour conduire au composé de formule (IV) : 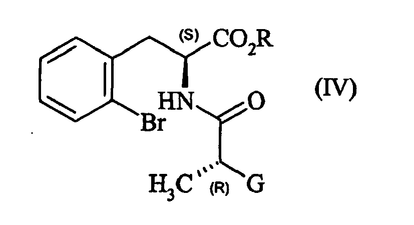 dans laquelle R et G sont tels que définis précédemment,
que l'on soumet à une réaction de couplage intramoléculaire, pour conduire au composé de formule (V) : 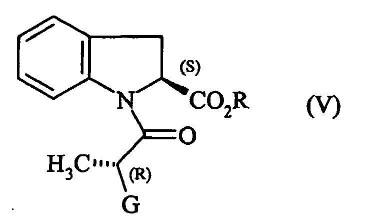 dans laquelle R et G sont tels que définis précédemment,
que l'on met en réaction avec le composé de formule (VI) : 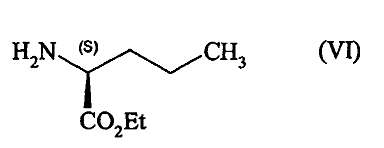 pour conduire au composé de formule (VII) : 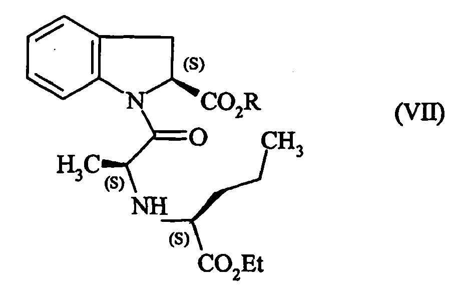 dans laquelle R est tel que défini précédemment,
que l'on soumet à une réaction d'hydrogénation catalytique, pour conduire, après déprotection le cas échéant, au composé de formule (I).

2. Procédé de synthèse selon la revendication 1, **caractérisé en ce que** R représente un groupement benzyle ou alkyle (C₁-C₆) linéaire ou ramifié.

3. Procédé de synthèse selon la revendication 1, **caractérisé en ce que** la réaction de couplage intramoléculaire est effectuée, soit en présence d'une base et d'un catalyseur à base de palladium, soit à l'aide d'hydrure de sodium et d'iodure de cuivre (I) ou de bromure de cuivre (I).

4. Procédé de synthèse selon la revendication 3, **caractérisé en ce que** la réaction de couplage intramoléculaire est effectuée en présence d'une base et d'un catalyseur à base de palladium et d'une arylphosphine ou d'une bis-phosphine.

5. Procédé de synthèse selon la revendication 4, **caractérisé en ce que** la base utilisée pour la réaction de couplage intramoléculaire est choisie parmi Cs₂CO₃, NaOtBu, Na₂CO₃, NaOAc et KOAc.

6. Procédé de synthèse selon la revendication 4 ou 5, **caractérisé en ce que** le catalyseur à base de palladium et d'une arylphosphine ou d'une bis-phosphine est choisi parmi Pd(0)/PPh₃, Pd(0)/P(*o*-tolyl)₃, Pd(0)/P(1-naphtyl)₃, Pd(0)/P(*o*-méthoxyphényl)₃, Pd₂(dba)₃/PPh₃, Pd₂(dba)₃/P(*o*-tolyl)₃, Pd₂(dba)₃/P(1-naphtyl)₃, Pd₂(dba)₃/P(*o-*méthoxyphényl)₃, Pd₂(dba)₃/P(2-furyl)₃, Pd₂(dba)₃/dppp, Pd₂(dba)₃/(±)-BINAP et (DPPF)PdCl₂.CH₂Cl₂/DPPF,
étant entendu que par BINAP, on entend 2,2'-bis(diphénylphosphino)-1,1'-binaphtyle, par dba, on entend dibenzylidèneacétone,
par DPPF, on entend 1,1'-bis(diphénylphosphino)ferrocène,
et par dppp, on entend 1,3-bis(diphénylphosphino)propane.

7. Procédé de synthèse selon la revendication 1, **caractérisé en ce que** G représente un atome de chlore ou de brome, ou un groupement p-toluènesulfonyloxy, méthanesulfonyloxy ou trifluorométhanesulfonyloxy.

8. Procédé de synthèse selon la revendication 7, **caractérisé en ce que** la réaction entre les composés de formules (V) et (VI) est effectuée en présence d'une amine organique choisie parmi la triéthylamine, la pyridine et la diisopropyléthylamine, ou une base minérale choisie parmi Na₂CO₃, K₂CO₃, NaHCO₃ et KHCO₃.

9. Procédé de synthèse selon la revendication 1, **caractérisé en ce que** G représente un groupement hydroxy.

10. Procédé de synthèse selon la revendication 9, **caractérisé en ce que** la réaction entre les composés de formules (V) et (VI) est effectuée en présence d'iodure de N-méthyl-N-phényl-aminotriphénylphosphonium, ou, lorsque R est différent de l'atome de hydrogène, par réaction de Mitsunobu.

11. Composé de formule (IV) : 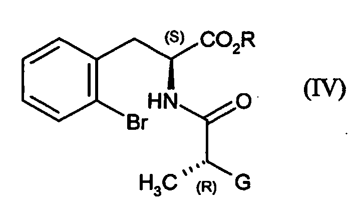 dans laquelle R et G sont tels que définis dans la revendication 1.

12. Procédé de synthèse selon l'une quelconque des revendications 1 à 10 du perindopril sous sa forme de sel de tert-butylamine.

## Claims

1. Process for the synthesis of perindopril of formula (I) : 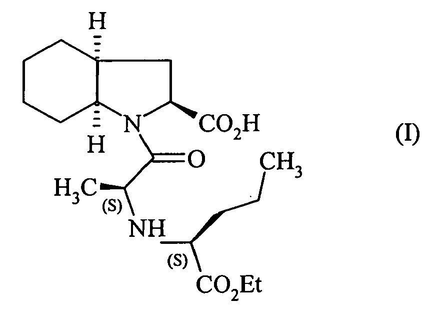 and pharmaceutically acceptable salts thereof,
**characterised in that** the compound of formula (II), of configuration (S) : 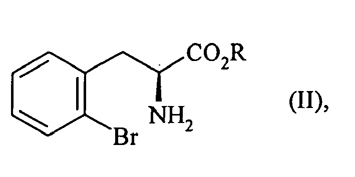 wherein R represents a hydrogen atom or a protecting group for the acid function,
is reacted with a compound of formula (III), of configuration (R) : 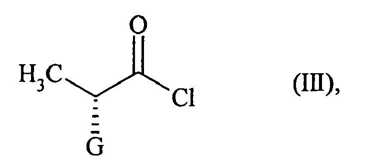 wherein G represents a chlorine or bromine atom or a hydroxy, p-toluenesulphonyloxy, methanesulphonyloxy or trifluoromethanesulphonyloxy group,
in the presence of a base
to yield the compound of formula (IV): 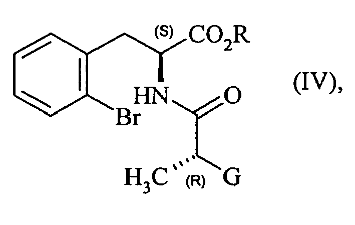 wherein R and G are as defined hereinbefore,
which is subjected to an intramolecular coupling reaction to yield the compound of formula (V) : 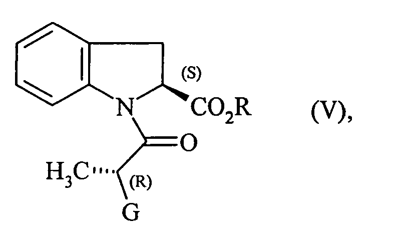 wherein R and G are as defined hereinbefore,
which is reacted with the compound of formula (VI) : 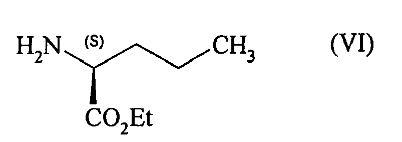 to yield the compound of formula (VII) : 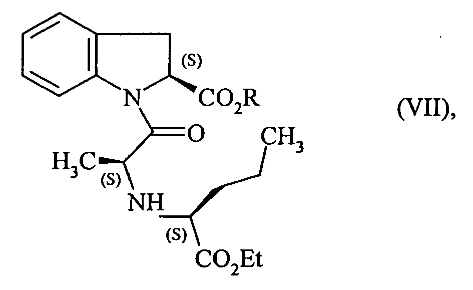 wherein R is as defined hereinbefore,
which is subjected to a catalytic hydrogenation reaction to yield, after deprotection where appropriate, the compound of formula (I).

2. Synthesis process according to claim 1, **characterised in that** R represents a benzyl or linear or branched (C₁-C₆)alkyl group.

3. Synthesis process according to claim 1, **characterised in that** the intramolecular coupling reaction is carried out either in the presence of a base and a catalyst based on palladium or using sodium hydride and copper(I) iodide or copper(I) bromide.

4. Synthesis process according to claim 3, **characterised in that** the intramolecular coupling reaction is carried out in the presence of a base and a catalyst based on palladium and on an arylphosphine or a bisphosphine.

5. Synthesis process according to claim 4, **characterised in that** the base used for the intramolecular coupling reaction is selected from Cs₂CO₃, NaOtBu, Na₂CO₃, NaOAc and KOAc.

6. Synthesis process according to claim 4 or 5, **characterised in that** the catalyst based on palladium and on an arylphosphine or a bisphosphine is selected from Pd(0)/PPh₃, Pd(0)/P(*o*-tolyl)₃, Pd(0)/P(1-naphthyl)₃, Pd(0)/P(*o*-methoxyphenyl)₃, Pd₂(dba)₃/PPh₃, Pd₂(dba)₃/P(*o*-tolyl)₃, Pd₂(dba)₃/P(1-naphthyl)₃, Pd₂(dba)₃/P(*o*-methoxyphenyl)₃, Pd₂(dba)₃/P(2-furyl)₃, Pd₂(dba)₃/dppp, Pd₂(dba)₃/(±)-BINAP and (DPPF)PdC1₂.CH₂C1₂/DPPF,
BINAP being understood to be 2,2'-bis(diphenylphosphino)-1,1'-binaphthyl, dba being understood to be dibenzylideneacetone,
DPPF being understood to be 1,1'-bis(diphenylphosphino)ferrocene
and dppp being understood to be 1,3-bis(diphenylphosphino)propane.

7. Synthesis process according to claim 1, **characterised in that** G represents a chlorine or bromine atom or a p-toluenesulphonyloxy, methanesulphonyloxy or trifluoromethanesulphonyloxy group.

8. Synthesis process according to claim 7, **characterised in that** the reaction between the compounds of formulae (V) and (VI) is carried out in the presence of an organic amine selected from triethylamine, pyridine and diisopropylethylamine or a mineral base selected from Na₂CO₃, K₂CO₃, NaHCO₃ and KHCO₃.

9. Synthesis process according to claim 1, **characterised in that** G represents a hydroxy group.

10. Synthesis process according to claim 9, **characterised in that** the reaction between the compounds of formulae (V) and (VI) is carried out in the presence of N-methyl-N-phenyl-aminotriphenylphosphonium iodide or, when R is other than a hydrogen atom, by a Mitsunobu reaction.

11. Compound of formula (IV) : 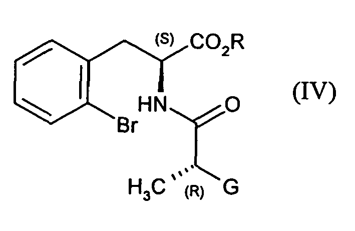 wherein R and G are as defined in claim 1.

12. Process according to any one of claims 1 to 10 for the synthesis of perindopril in the form of its tert-butylamine salt.

## Patentansprüche

1. Verfahren zur Synthese von Perindopril der Formel (I): 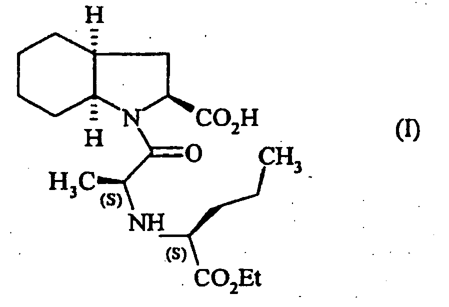 und von seinen pharmazeutisch annehmbaren Salzen,
**dadurch gekennzeichnet, daß** man die Verbindung der Formel (II) in der Konfiguration (S): 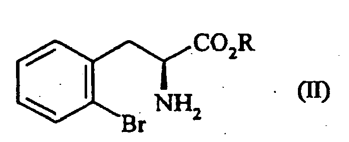 in der R ein Wasserstoffatom oder eine Schutzgruppe für die Säurefunktion bedeutet,
mit einer Verbindung der Formel (III) in der Konfiguration (R): 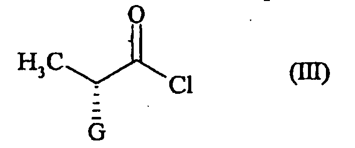 in der G ein Chlor- oder Bromatom oder eine Hydroxy-, p-Toluolsulfonyloxy-, Methansulfonyloxy- oder Trifluormethansulfonyloxy-gruppe bedeutet,
in Gegenwart einer Base umsetzt,
zur Bildung der Verbindung der Formel (IV): 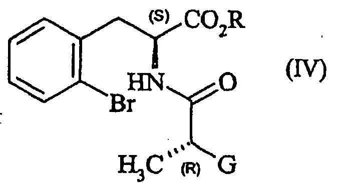 in der R und G die oben angegebenen Bedeutungen besitzen,
welche man einer intramolekularen Kupplungsreaktion unterwirft zur Bildung der Verbindung der Formel (V): 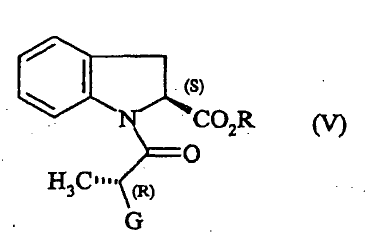 in der R und G die oben angegebenen Bedeutungen besitzen,
welche man mit der Verbindung der Formel (VI): 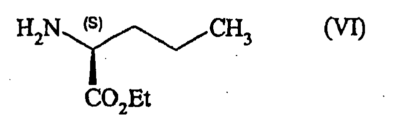 umsetzt zur Bildung der Verbindung der Formel (VII): 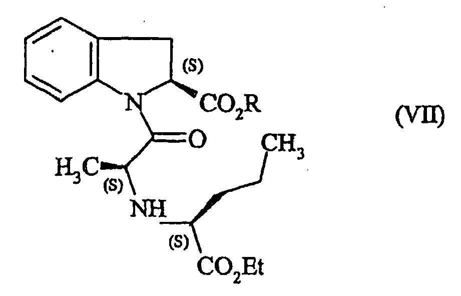 in der R die oben angegebenen Bedeutungen besitzt,
welche man einer katalytischen Hydrierungsreaktion unterwirft, so daß man gegebenenfalls nach der Abspaltung der Schutzgruppe die Verbindung der Formel (I) erhält.

2. Syntheseverfahren nach Anspruch 1, **dadurch gekennzeichnet, daß** R eine Benzylgruppe oder eine geradkettige oder verzweigte (C₁-C₆)-Alkylgruppe bedeutet.

3. Syntheseverfahren nach Anspruch 1, **dadurch gekennzeichnet, daß** die intramolekulare Kupplungsreaktion entweder in Gegenwart einer Base und eines Katalysators auf der Grundlage von Palladium oder mit Hilfe von Natriumhydrid und Kupfer(I)-iodid oder Kupfer(I)-bromid durchgeführt wird.

4. Syntheseverfahren nach Anspruch 3, **dadurch gekennzeichnet, daß** die intramolekulare Kupplungsreaktion in Gegenwart einer Base, eines Katalysators auf der Grundlage von Palladium und eines Arylphosphins oder eines Bisphosphins durchgeführt wird.

5. Syntheseverfahren nach Anspruch 4, **dadurch gekennzeichnet, daß** die für die intramolekulare Kupplungsreaktion verwendete Base ausgewählt wird aus Cs₂CO₃, NaOtBu, Na₂CO₃, NaOAc und KOAc.

6. Syntheseverfahren nach Anspruch 4 oder 5, **dadurch gekennzeichnet, daß** der Katalysator auf der Grundlage von Palladium und das Arylphosphin oder Bisphosphin ausgewählt werden aus Pd(0)/PPh₃, Pd(0)/P(*o*-Tolyl)₃, Pd(0)/P(1-Naphthyl)₃, Pd(0)/P(o-Methoxyphenyl)₃, Pd₂(dba)₃/PPh₃, Pd₂(dba)₃/P(*o*-Tolyl)₃, Pd₂(dba)₃/P(1-Naphthyl)₃, Pd₂(dba)₃/P(*o*-Methoxyphenyl)₃, Pd₂(dba)₃/P(2-Furyl)₃, Pd₂(dba)₃/dppp, Pd₂(dba)₃/(±)-BINAP und (DPPD)PdCl₂·CH₂Cl₂/DPPF,
mit der Maßgabe, daß man unter BINAP 2,2'-Bis(diphenylphosphin)-1,1'-binaphthyl,
unter dba Dibenzylidenaceton,
unter DPPF 1,1'-Bis(diphenylphosphino)-ferrocen und
und dppp 1,3-Bis(diphenylphosphino)-propan versteht.

7. Syntheseverfahren nach Anspruch 1, **dadurch gekennzeichnet, daß** G ein Chlor- oder Bromatom oder eine p-Toluolsulfonyloxygruppe, Methansulfonyloxygruppe oder Trifluormethansulfonyloxygruppe bedeutet.

8. Syntheseverfahren nach Anspruch 7, **dadurch gekennzeichnet, daß** die Reaktion der Verbindungen der Formeln (V) und (VI) in Gegenwart eines organischen Amins ausgewählt aus Triethylamin, Pyridin und Diisopropylethylamin oder einer anorganischen Base ausgewählt aus Na₂CO₃, K₂CO₃, NaHO₃ und KHCO₃ durchgeführt wird.

9. Syntheseverfahren nach Anspruch 1, **dadurch gekennzeichnet, daß** G eine Hydroxygruppe bedeutet.

10. Syntheseverfahren nach Anspruch 9, **dadurch gekennzeichnet, daß** die Reaktion der Verbindungen der Formel (V) und (VI) in Gegenwart von N-Methyl-N-phenyl-aminotriphenylphosphoniumiodid oder, wenn R von einem Wasserstoffatom verschieden ist, durch eine Mitsunobu-Reaktion erfolgt.

11. Verbindung der Formel (IV): 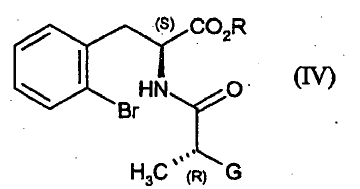 in der R und G die in Anspruch 1 angegebenen Bedeutungen besitzen.

12. Syntheseverfahren nach einem der Ansprüche 1 bis 10 für die Herstellung von Perindopril in Form seines tert.-Butylaminsalzes.
